# FASCICULE DE BREVET EUROPEEN

(11) **EP 1 062 924 B1**
(45) Date de publication et mention de la délivrance du brevet: **06.05.2004**
(21) Numéro de dépôt: 99420142.4
(22) Date de dépôt: 22.06.1999
(51) Int. Cl.: A61F 2/42

(54) **Prothèse d'articulation de poignet**
Handgelenkprothese
Wrist joint prosthesis

(43) Date de publication de la demande: 27.12.2000
(73) Titulaire: Biomet Merck France, 26000 Valence (FR); Isselin, Jacques, 21560 Couternon (FR)
(72) Inventeur: Isselin, Jacques, 21560 Couteron (FR); De Witte, Gérard, 26300 Chateauneuf sur Isere (FR)
(74) Mandataire: Vuillermoz, Bruno

(56) Documents cités:
- EP-A- 0 500 477
- EP-A- 0 532 440
- EP-A- 0 607 748
- EP-A- 0 607 749
- FR-A- 2 579 454
- FR-A- 2 673 100
- FR-A- 2 773 060
- GB-A- 2 032 782
- US-A- 3 896 503
- US-A- 4 106 128
- US-A- 4 164 793
- US-A- 4 259 752

## Description

L'invention concerne une prothèse d'articulation du poignet.

L'articulation du poignet est constituée par les os du carpe, lesquels se présentent sous forme de deux rangées intercalées entre le radius et les os métacarpiens. Chacun des os du carpe présente des surfaces articulaires complémentaires permettant l'articulation du poignet proprement dit.

Une fracture d'un os du carpe mais également le vieillissement de ces os, lequel se traduit par un phénomène d'arthrose, conduit à détériorer, voire même détruire les surfaces articulaires.

Le traitement de l'arthrose consiste, dans un premier temps, à pallier la douleur à l'aide d'anti-inflammatoires.

Lorsque la douleur devient trop importante, on a alors recours à la mise en place d'une prothèse d'articulation. Si les os de la première rangée du carpe, c'est à dire le scaphoïde, le semi lunaire, le pyramidal et le pisiforme sont trop détériorés, on procède à leur ablation, avant la mise en place de la prothèse.

Dans le document FR-A-2 673 100, on a décrit une prothèse constituée d'un élément dit capital, lequel se présente sous la forme d'une tige conique à l'extrémité de laquelle est fixée, de façon amovible, une tête sous forme d'une calotte sphérique. La tige conique est destinée à être ancrée dans le grand os, également appelé capitatum, appartenant aux os constitutifs de la seconde rangée du carpe. La calotte sphérique constitue une surface articulaire convexe, apte à prendre appui contre une pièce implantée dans le radius. Cette pièce est constituée d'une embase fixée sur un plateau, lequel est rapporté à l'extrémité libre d'une tige tronconique préalablement implantée dans le canal médullaire du radius. L'élément capital est mis en place après résection de la tête du grand os, de sorte à délimiter un plan susceptible de recevoir la face plane de la tête. De plus et en pratique, compte tenu de la forme sphérique de la calotte, il est nécessaire d'effectuer non seulement la résection du grand os mais également celle de l'os crochu voisin. En outre, on note que le plateau n'est pas parfaitement en appui sur le radius, ce qui amoindrit sa stabilité.

Par ailleurs, la forme de la calotte affecte la gaine tendineuse passant au niveau du grand os et de l'os crochu.

Autrement dit, la mise en place d'une telle prothèse nécessite des opérations complémentaires de résection, lesquelles augmentent non seulement la durée de l'intervention mais surtout obligent à fragiliser certain os (le grand os et l'os crochu) qui peuvent pourtant être en bon état.

Le problème que se propose de résoudre l'invention est donc de fournir une prothèse qui puisse être mise en place sans résection préalable du grand os ni de l'os crochu et permettant de sauvegarder dans le même temps, l'intégrité et le fonctionnement de l'os crochu.

Pour ce faire, l'invention propose une prothèse d'articulation du poignet constituée d'au moins deux éléments, respectivement une pièce d'articulation destinée à être ancrée au niveau de l'extrémité proximale du grand os et une pièce d'appui destinée à être ancrée dans l'extrémité distale du radius après résection partielle de celle-ci, les deux éléments étant aptes à coopérer entre eux de sorte à permettre l'articulation du poignet.

Cette prothèse se caractérise en ce que ladite pièce d'articulation se présente sous la forme d'une calotte sphérique tronquée, monobloc, munie d'une tige, issue du fond concave de ladite calotte, et destinée à être implantée dans le grand os, de telle sorte à ce que ladite calotte vienne coiffer le grand os.

En d'autres termes, l'invention consiste à avoir réalisé une prothèse dont la pièce d'articulation présente une forme particulière, à savoir une calotte sphérique tronquée, laquelle est creuse et interrompue, de sorte à pouvoir épouser la forme de l'extrémité proximale du grand os en combinaison avec celle de l'os crochu latéral.

Selon une première caractéristique de l'invention, la tige est implantée dans le grand os, sans résection préalable de celui-ci, ce qui permet non seulement de simplifier l'opération chirurgicale mais également et surtout de sauvegarder l'intégrité et le bon fonctionnement du grand os et de l'os crochu.

Pour que la pièce d'articulation soit fixée avec une efficacité optimale, la tige est confondue avec l'axe de révolution de la calotte. Elle peut donc être insérée sur toute sa longueur à l'extrémité proximale du grand os, et ainsi coiffer ladite extrémité.

De plus et en pratique, la troncature de la calotte sphérique est réalisée selon un plan parallèle au plan séparant le grand os de l'os crochu et positionnée de manière adjacente à celui-ci, permettant ainsi de sauvegarder l'articulation entre le grand os et de l'os crochu, et partant de conserver les mouvements naturels de cette articulation.

Pour permettre l'articulation effective du poignet, la pièce d'appui comporte une embase destinée d'une part à être insérée au niveau de l'extrémité distale du radius et d'autre part à recevoir un plateau de friction présentant une forme concave en direction du grand os, ledit plateau étant destiné à coopérer avec ladite calotte sphérique tronquée constitutive de la pièce d'articulation.

Pour permettre la fixation de l'embase au niveau de l'extrémité distale du radius, après résection de celle-ci, l'embase est plane et comporte un pion de forme cylindrique destinée à coopérer avec un orifice ménagé à cet effet au niveau de l'extrémité distale du radius.

Autrement dit, l'embase n'est pas rapportée sur une tige préalablement implantée, mais au contraire se présente monobloc, de sorte à être en appui sur toute sa surface au niveau de l'extrémité distale du radius, assurant ainsi une stabilité optimale.

Avantageusement, pour éviter toute rotation de l'embase après implantation dans le radius, celle-ci comporte en outre deux pointes parallèles dirigées en direction du radius, et ménagées au niveau de sa bordure latérale.

En outre et selon une autre caractéristique de l'invention, le pion est percé d'un orifice débouchant permettant le passage et la retenue d'une vis.

Par ailleurs, pour permettre la repousse osseuse au niveau de l'extrémité distale du radius, la face inférieure de l'embase présente des stries radiales aptes à favoriser la fixation secondaire.

De plus, pour permettre la fixation du plateau sur l'embase, la face inférieure du plateau présente une protubérance destinée à coopérer avec l'orifice traversant du pion cylindrique de l'embase.

En outre, pour éviter tout jeu du plateau par rapport à l'embase, cette dernière comporte sur au moins l'une de ses bordures latérales, une saillie destinée à coopérer avec une échancrure complémentaire ménagée sur le bord latéral correspondant du plateau. En pratique, la coopération du plateau sur l'embase s'effectue par enclipsage ou encliquetage.

L'ensemble constitué par le plateau et l'embase peut être monobloc, et réalisé en métal, polyéthylène, voire céramique.

L'invention et les avantages qui en découlent ressortiront mieux de l'exemple de réalisation suivant à l'appui des figures annexées.

La figure 1 est une vue éclatée en perspective de la prothèse d'articulation selon l'invention.

La figure 2 est une vue analogue de la figure 1 selon une autre orientation.

La figure 3 est une vue latérale de la prothèse assemblée.

La figure 4 est une vue de la prothèse analogue à la figure 3, selon l'angle opposé.

La figure 5 illustre la mise en place de la prothèse au sein de l'articulation du poignet.

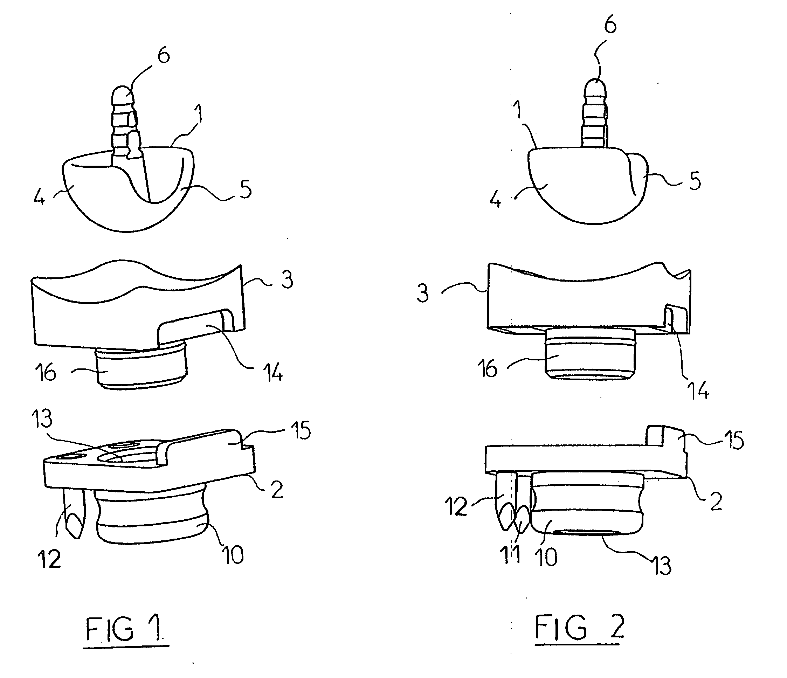

Comme le montrent les figures 1 et 2, la prothèse selon l'invention est constituée d'au moins deux éléments, respectivement une pièce d'articulation (1) et une pièce d'appui (2, 3).

La pièce d'articulation (1) se présente sous la forme d'une calotte sphérique (4) tronquée (5) monobloc, munie d'une tige (6) issue du fond concave de ladite calotte. La tige (6) est positionnée de telle sorte à ce qu'elle se confonde avec l'axe de révolution de la calotte (4).

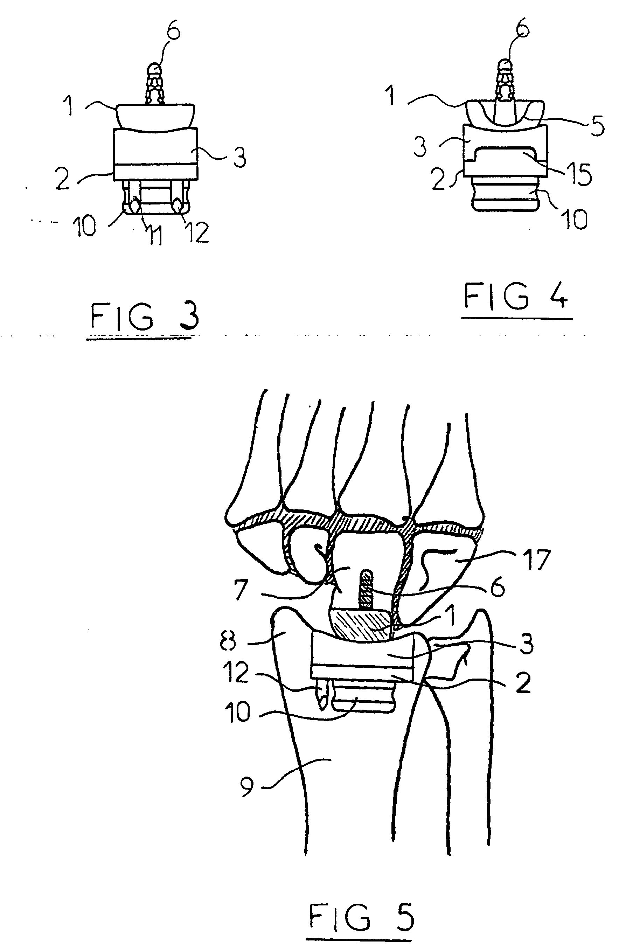

En pratique et comme le montre la figure 5, cette calotte est destinée à être implantée au niveau du pôle supérieur du grand os (7), de sorte à venir coiffer celle-ci. En d'autres termes, aucune résection du grand os n'est nécessaire pour la mise en place de la calotte.

De plus, la troncature (5) est réalisée selon un plan parallèle au plan séparant le grand os (7) de l'os crochu (17) et positionnée de manière adjacente à celui-ci.

La pièce d'appui est constituée d'une embase (2) et d'un plateau (3). L'embase (2) est destinée à être insérée dans l'extrémité (8) du radius (9) après résection de celle-ci.

Cette embase est plane et comporte sur sa face inférieure un pion (10) de forme cylindrique destinée à coopérer avec un orifice correspondant, ménagé dans l'extrémité distale du radius. De façon connue, cet orifice est réalisé par alésage et le pion y est introduit en force.

En outre, afin d'éviter la rotation de l'embase (2) sur l'extrémité distale du radius, le bord latéral de celle-ci présente deux pointes parallèles (11, 12) destinées à être ancrées dans le radius.

Selon la forme de réalisation représentée sur les figures, ces deux pointes présentent la même longueur que celle du pion.

Bien entendu, on peut prévoir tout autre moyen équivalent aux pointes et notamment une saillie.

De même, pour favoriser la repousse osseuse, des stries radiales (non représentées) sont ménagées sur la face inférieure de l'embase.

Selon une forme de réalisation avantageuse, le pion cylindrique (10) est percée d'un orifice débouchant (13) permettant le passage d'une vis de fixation laquelle est vissée dans un orifice préalablement effectué par taraudage dans l'extrémité du radius.

L'orifice débouchant a également pour fonction de recevoir une protubérance (16) correspondante ménagée sur la face inférieure du plateau, permettant ainsi la fixation des deux éléments. Parallèlement, la face supérieure du plateau présente une forme concave en direction du grand os apte à coopérer avec la pièce d'articulation.

De plus, le plateau présente sur sa face latérale une échancrure (14) destinée à coopérer avec une saillie (15) ménagée sur le bord latéral correspondant de l'embase (2), permettant d'éviter ainsi tout jeu du plateau sur l'embase. La coopération du plateau sur l'embase est réalisée par enclipsage ou encliquetage.

Par ailleurs, le plateau est en pratique réalisé en polyéthylène. On peut néanmoins concevoir sa réalisation en céramique, voire en métal.

Lorsque l'on désire mettre en place cette prothèse, on procède tout d'abord à l'ablation partielle de la première rangée de carpe, c'est à dire à l'ablation du scaphoïde, du semi lunaire et du pyramidal, le pisiforme étant conservé. On résèque ensuite l'extrémité distale du radius puis on effectue plusieurs orifices par alésage et/ou taraudage, susceptibles de recevoir le pion (10) de l'embase, les pointes (11, 12) mais également la vis de serrage. Comme déjà dit, le pion cylindrique et les pointes de l'embase sont introduits en force dans l'alésage.

Il suffit ensuite positionner le plateau sur l'embase en faisant coopérer non seulement la protubérance (16) dans l'orifice débouchant (14) mais également l'échancrure (14) et la saillie (13) par enclipsage ou encliquetage.

Par ailleurs, on effectue un taraudage au niveau de l'extrémité proximale ou de la tête du grand os (17) sans aucune résection de celui-ci, puis on insère la tige (6) de la pièce d'articulation.

En pratique, la tige (6) de la calotte sphérique est de forme conique et présente des échancrures destinées à assurer une fixation efficace de celle-ci au sein du grand os (17). Comme déjà dit, cette calotte sphérique est tronquée, la zone tronquée étant dirigée en direction de l'os crochu (17). De la sorte il est inutile de réséquer non seulement le grand os mais également l'os crochu.

Les avantages de l'invention ressortent bien de la description.

On notera en particulier, la simplicité de mise en place, la stabilité de la prothèse implantée mais également l'absence de toute résection préalable du grand os et de l'os crochu, et enfin la préservation des mouvements naturels de l'articulation.

## Revendications

1. Prothèse d'articulation du poignet constituée d'au moins deux éléments, respectivement une pièce d'articulation (1) destinée à être ancrée au niveau de l'extrémité proximale du grand os (7) et une pièce d'appui (2, 3) destinée à être ancrée dans l'extrémité distale (8) du radius (9) après résection partielle de celle-ci, les deux éléments étant aptes à coopérer entre eux de sorte à permettre l'articulation du poignet, ***caractérisée* en ce que** ladite pièce d'articulation (1) se présente sous la forme d'une calotte (4) sphérique tronquée (5), monobloc, munie d'une tige (6), issue du fond concave de ladite calotte (4), et destinée à être implantée dans le grand os (7), de telle sorte à ce que ladite calotte vienne coiffer le grand os.

2. Prothèse d'articulation du poignet selon la revendication 1, ***caractérisée* en ce que** la tige (6) est confondue avec l'axe de révolution de la calotte (4).

3. Prothèse d'articulation du poignet selon l'une des revendications 1 et 2, ***caractérisée* en ce que** la troncature (5) est réalisée selon un plan parallèle au plan séparant le grand os de l'os crochu et positionnée de manière adjacente à celui-ci.

4. Prothèse d'articulation du poignet selon l'une des revendications 1 à 3, ***caractérisée* en ce que** la pièce d'appui comporte une embase (2) destinée, d'une part à être insérée au niveau de l'extrémité distale (8) du radius (9), et d'autre part à recevoir un plateau (3) de friction présentant une forme concave en direction du grand os (7), ledit plateau étant destiné à coopérer avec ladite calotte (4) sphérique tronquée (5) constitutive de la pièce d'articulation (1).

5. Prothèse d'articulation du poignet selon la revendication 4, ***caractérisée* en ce que** l'embase (2) est plane et comporte un pion (10) de forme cylindrique destiné à coopérer avec un orifice ménagé à cet effet au niveau de l'extrémité distale (8) du radius (9).

6. Prothèse d'articulation du poignet selon la revendication 5, ***caractérisée* en ce que** l'embase (2) comporte en outre deux pointes parallèles (11, 12) dirigées en direction du radius et ménagées au niveau la bordure latérale de ladite embase.

7. Prothèse d'articulation du poignet selon la revendication 5, ***caractérisée* en ce que** le pion est percé d'un orifice traversant (13) permettant le passage, puis la retenue d'une vis.

8. Prothèse d'articulation du poignet selon l'une des revendications 4 à 7, ***caractérisée* en ce que** la face inférieure de l'embase (2) présente des stries radiales aptes à favoriser la fixation secondaire.

9. Prothèse d'articulation du poignet selon l'une des revendications 4 à 8, ***caractérisée* en ce que** l'embase (2) comporte sur au moins l'une de ses bordures latérales, une saillie (15) destinée à coopérer avec une échancrure (14) complémentaire ménagée sur le bord latéral correspondant du plateau (3).

10. Prothèse d'articulation du poignet selon l'une des revendications 4 à 9, ***caractérisée* en ce que** la face inférieure du plateau (3) présente une protubérance (16) destinée à coopérer avec l'orifice (13) traversant du pion cylindrique (10) de l'embase (2).

## Patentansprüche

1. Handgelenksprothese, bestehend aus mindestens zwei Elementen, nämlich einem Gelenkteil (1), welches dazu bestimmt ist, im Bereich des proximalen Endes des Kopfbeins (7) verankert zu werden, und einem Stützteil (2, 3), welches dazu bestimmt ist, im distalen Ende (8) des Radius (9) nach einer partiellen Resektion dieses distalen Endes verankert zu werden, wobei die zwei Elemente dazu geeignet sind, miteinander zusammen zu wirken, um die Gelenkverbindung des Handgelenks zu ermöglichen, **dadurch gekennzeichnet, daß** das genannte Gelenkteil (1) die Form einer abgeschnittenen (5) sphärischen Kalotte (4) aus einem Stück aufweist, welche mit einem Schaft (6) versehen ist, der vom konkaven Grund der genannten Kalotte (4) ausgeht und dazu bestimmt ist, in das Kopfbein (7) implantiert zu werden, so daß die genannte Kalotte das Kopfbein überdeckt.

2. Handgelenksprothese nach Anspruch 1, **dadurch gekennzeichnet, daß** der Schaft (6) mit der Drehachse der Kalotte (4) zusammenfällt.

3. Handgelenksprothese nach einem der Ansprüche 1 und 2, **dadurch gekennzeichnet, daß** der Abschnitt (5) entlang einer Ebene durchgeführt ist, die parallel zur Ebene verläuft, welche das Kopfbein vom Hakenbein trennt, und dieser benachbart positioniert ist.

4. Handgelenksprothese nach einem der Ansprüche 1 bis 3, **dadurch gekennzeichnet, daß** das Stützteil einen Sockel (2) umfaßt, der dazu bestimmt ist, einerseits im Bereich des distalen Endes (8) des Radius (9) eingefügt zu werden und andererseits eine Gleitplatte (3) aufzunehmen, welche eine konkave Form in Richtung des Kopfbeins (7) aufweist, wobei die genannte Platte dazu bestimmt ist, mit der genannten abgeschnittenen (5), sphärischen Kalotte (4), welche das Gelenkteil (1) bildet, zusammen zu wirken.

5. Handgelenksprothese nach Anspruch 4, **dadurch gekennzeichnet, daß** der Sockel (2) eben ist und ein Fußstück (10) mit zylindrischer Form aufweist, welches dazu bestimmt ist, mit einer hierfür im Bereich des distalen Endes (8) des Radius (9) angebrachten Öffnung zusammen zu wirken.

6. Handgelenksprothese nach Anspruch 5, **dadurch gekennzeichnet, daß** der Sockel (2) ferner zwei parallele Spitzen (11, 12) aufweist, die in Richtung des Radius weisen und im Bereich der seitlichen Umrandung des genannten Sockels angebracht sind.

7. Handgelenksprothese nach Anspruch 5, **dadurch gekennzeichnet, daß** das Fußstück von einer durchgehenden Öffnung (13) durchragt wird, welche das Hindurchtreten und anschließend den Halt einer Schraube ermöglicht.

8. Handgelenksprothese nach einem der Ansprüche 4 bis 7, **dadurch gekennzeichnet, daß** die untere Seite des Sockels (2) radiale Rillen aufweist, die dazu geeignet sind, die Sekundärfixation zu fördern.

9. Handgelenksprothese nach einem der Ansprüche 4 bis 8, **dadurch gekennzeichnet, daß** der Sockel (2) mindestens auf einer seiner seitlichen Umrandungen einen Vorsprung (15) aufweist, der dazu bestimmt ist, mit einer komplementären Aussparung (14) zusammen zu wirken, welche an dem entsprechenden seitlichen Rand der Platte (3) angebracht ist.

10. Handgelenksprothese nach einem der Ansprüche 4 bis 9, **dadurch gekennzeichnet, daß** die untere Seite der Platte (3) eine Ausstülpung (16) aufweist, welche dazu bestimmt ist, mit der Öffnung (13), die das zylindrische Fußstück (10) des Sockels (2) durchragt, zusammen zu wirken.

## Claims

1. Wrist joint prosthesis consisting of at least two elements, namely an articulation component (1) intended to be anchored at the proximal end of the capitate bone (7), and a bearing component (2, 3) intended to be anchored in the distal end (8) of the radius (9) after partial resection thereof, the two elements being able to cooperate with one another so as to permit articulation of the wrist, **characterized in that** said articulation component (1) is in the form of a one-piece spherical cap (4) which is truncated (5) and is equipped with a stem (6) issuing from the concave base of said cap (4) and intended to be implanted in the capitate bone (7) in such a way that said cap covers the end of the capitate bone.

2. Wrist joint prosthesis according to Claim 1, **characterized in that** the stem (6) coincides with the axis of revolution of the cap (4).

3. Wrist joint prosthesis according to either of Claims 1 and 2, **characterized in that** the truncation (5) is formed in a plane parallel to the plane separating the capitate bone from the hamate bone and is positioned so as to be adjacent thereto.

4. Wrist joint prosthesis according to one of Claims 1 to 3, **characterized in that** the bearing component comprises a seat (2) intended, on the one hand, to be inserted at the distal end (8) of the radius (9) and, on the other hand, to receive a friction plateau (3) having a shape concave in the direction of the capitate bone (7), said plateau being intended to cooperate with said truncated (5) spherical cap (4) constituting the articulation component (1).

5. Wrist joint prostheis according to Claim 4, **characterized in that** the seat (2) is flat and comprises a plug (10) of cylindrical shape intended to cooperate with an orifice formed for this purpose in the distal end (8) of the radius (9).

6. Wrist joint prosthesis according to Claim 5, **characterized in that** the seat (2) additionally comprises two parallel spikes (11, 12) which are oriented in the direction of the radius and are formed at the lateral margin of said seat.

7. Wrist joint prosthesis according to Claim 5, **characterized in that** the plug is provided with a through-orifice (13) permitting passage, then retention, of a screw.

8. Wrist joint prosthesis according to one of Claims 4 to 7, **characterized in that** the lower face of the seat (2) has radial striations which are able to promote secondary fixation.

9. Wrist joint prosthesis according to one of Claims 4 to 8, **characterized in that** the seat (2) comprises, on at least one of its lateral margins, a projection (15) intended to cooperate with a complementary indent (14) formed on the corresponding lateral edge of the plateau (3).

10. Wrist joint prosthesis according to one of Claims 4 to 9, **characterized in that** the lower face of the plateau (3) has a protuberance (16) intended to cooperate with the through-orifice (13) of the cylindrical plug (10) of the seat (2).
